# EUROPÄISCHE PATENTSCHRIFT

(11) **EP 1 962 802 B1**
(45) Veröffentlichungstag und Bekanntmachung des Hinweises auf die Patenterteilung: **14.10.2015**
(21) Anmeldenummer: 06840985.3
(22) Anmeldetag: 15.12.2006
(51) Int. Cl.: A61L 15/46, A61L 15/60, A61L 15/18, A61K 33/38

(54) **MEDIZINISCHE ZUSAMMENSETZUNG MIT WIRKSTOFFEN SOWIE WUNDKONTAKTSCHICHT MIT EINER ZUSAMMENSETZUNG**
MEDICINAL COMPOSITION WITH ACTIVE INGREDIENTS AND WOUND CONTACT LAYER WITH A COMPOSITION
COMPOSITION MEDICINALE CONTENANT DES SUBSTANCES ACTIVES ET COUCHE EN CONTACT AVEC LA PLAIE COMPRENANT CETTE COMPOSITION

(30) Priorität: 17.12.2005 DE 102005060461
(43) Veröffentlichungstag der Anmeldung: 03.09.2008
(73) Patentinhaber: Paul Hartmann AG, 89522 Heidenheim (DE)
(72) Erfinder: EFFING, Jochem, 65779 Kelkheim-Fischbach (DE)
(86) Internationale Anmeldenummer: PCT/EP2006/012102
(87) Internationale Veröffentlichungsnummer: WO 2007/068492

(56) Entgegenhaltungen:
- EP-A- 1 535 605
- EP-A2- 1 159 972
- WO-A-96/36315
- WO-A-03/066001
- WO-A-2005/009403
- DE-A1-102004 031 955
- US-A- 4 503 034
- US-A- 5 681 579
- US-A1- 2005 281 762
- US-B1- 6 375 977
- US-B2- 6 794 555

## Beschreibung

Die vorliegende Erfindung betrifft eine medizinische Zusammensetzung und deren Verwendung zur Wundbehandlung.

Eine Vielzahl an Salben oder anderen Zusammensetzungen sind in den vergangenen Dekaden zur Behandlung am Menschen bekannt und eingesetzt geworden. Diese Salben sind meist halbfeste Zusammensetzungen, die zur Anwendung auf gesunder Haut oder einigen Schleimhäuten wie beispielsweise auf den Augen bestimmt sind. Meist soll durch diese Salben oder Zubereitungen eine lokale Wirkung ausgeübt, Wirkstoffe perkutan verabreicht oder eine erweichende oder schützende Wirkung auf die Haut ausgeübt werden.

Darüber hinaus sind auch zahlreiche Salben zur Wundversorgung bekannt. So beschreibt beispielsweise die EP 621 031 eine Wundsalbe, die als Gel formuliert ist und mindestens ein gelbildendes Polysaccharid und Hexyleneglycol umfasst. Als gelbildendes Polysaccharid soll insbesondere Carboxymethylcellulose oder Natriumalginat eingesetzt werden. Diese Zusammensetzung soll eine antimikrobielle Wirkung aufweisen und nicht toxisch in Bezug auf Fibroplasten sein.

Des Weiteren wird mit der EP 107 526 eine Paste zum Schutz der Haut beispielsweise bei der Wundbehandlung oder der Stomaversorgung beschrieben, die als Gel formuliert Polyvinylpyrrolidon, Carboxymethylcellulose, Alginat, Wasser, ein Öl und einen Fettsäureester umfasst. Dieses Gel enthält dabei mindestens 20 Gew.-% Wasser und mindestens 45 Gew.-% Hydrocolloide.

Darüber hinaus sind hydrophile Salben bekannt, die einen begrenzten Anteil an Wasser aufnehmen und zur Wundversorgung eingesetzt werden können. Diese Salben beinhalten ein Gemisch aus verschiedenen Mono-, Di- und Triglyceriden und einem unpolaren Öl und werden beispielsweise in den Produkten Atrauman® auf Trägermaterialien zur Herstellung von so genannten Salbenkompressen verarbeitet.

Weiterhin ist mit der EP 65 399 eine sterile Wundauflage bekannt, die ein mit einer Wundsalbe imprägniertes Trägermaterial und einen wasserlöslichen Film aus Polyvinylpyrrolidon aufweist. Die Salbe kann eine hydrophile oder hydrophobe Wundsalbe sein.

Mit der WO 96/ 036 315 ist eine sterilisierbare Paste oder Creme bekannt, die eine Emulsion und ein wasserunlösliches, gelbildendes Material umfasst, das vernetzte Carboxymethylcellulose sein kann. Die Emulsion ihrerseits soll ein Öl oder Wax, Wasser und einen Emulgator umfassen, wobei der Wassergehalt mindestens 40 Gew.-% beträgt.

Mit der WO 01/ 070 285 ist eine Kompresse zur Behandlung von Wunden bekannt, die eine hydrophobe Elastomer-Matrix umfasst, in die Hydrocolloidpartikel dispergiert sind. Die Matrix soll weiterhin 55 bis 90 Gew.-% eines unpolaren Öls und ein grenzflächenaktives Mittel mit einem HLB-Wert größer als 10 aufweisen.

Ausgehend vom Stand der Technik ist es eine Aufgabe der vorliegenden Erfindung, eine Zubereitung bereitzustellen, die in der Lage ist, von einer Wunde abgegebenes Wundexsudat aufzunehmen sowie den Heilungsprozess einer Wunde optimal zu unterstützen, indem ein Wirkstoff freigesetzt wird. Weiterhin soll eine Wundkontaktschicht bereitgestellt werden, die sowohl Wundexsudat aufnehmen als auch einen Wirkstoff an eine Wunde oder die Wunde umgebende Haut abgeben kann, wobei die Wirkstoffabgabe auch über einen längeren Zeitraum erfolgen soll. Darüber hinaus soll eine Wundkontaktschicht zur Verfügung gestellt werden, die in der Lage ist, von einer Wunde abgegebenes Wundexsudat aufzunehmen, einen Wirkstoff abzugeben und ein feuchtes Wundheilungsklima zur Verfügung zu stellen und somit eine Wundheilung optimal unterstützt.

Gelöst werden diese Aufgaben durch eine medizinische gemäß Anspruch 1.

Weiterhin werden diese Aufgaben gelöst durch eine Wundkontaktschicht, die eine solche Zusammensetzung und ein Trägermaterial für die Zusammensetzung umfasst. Hierbei und im Folgenden sind im Zusammenhang mit der vorliegenden Erfindung alle Gehaltsangaben als Gewichts-% in Bezug auf die Gesamtmasse der medizinischen Zusammensetzung zu verstehen, sofern nichts anderes angegeben ist. Des Weiteren soll im Zusammenhang mit der vorliegenden Erfindung als hydrophile Basis eine medizinische Zusammensetzung verstanden sein, die ein- oder mehrphasig ist und durch den Anteil an mindestens einem Emulgator als Emulsion vorliegt oder in der Lage ist eine Emulsion auszubilden. Bei solchen Emulsionen kann es sich um Emulsionen handeln, die mindestens eine Wasser- und/oder Gelphase und mindestens eine Ölphase umfassen.

Ein Vorteil dieser Zusammensetzung besteht darin, dass diese Zusammensetzung durch den Gehalt an Hydrocolloiden, die in der hydrophilen Basis dispergiert sind, eine besonders große Menge an Flüssigkeiten wie beispielsweise Wundexsudat sehr schnell aufnehmen kann. Die hydrophile Basis bildet dabei bei Kontakt mit Flüssigkeiten innerhalb kurzer Zeit eine Emulsion aus, worauf in einem zweiten Schritt das Wasser in der Emulsion von den in der Basis dispers verteilten Hydrocolloiden aufgenommen wird. Dieser Vorgang kann auch parallel erfolgen. In jedem Fall bilden die Hydrocolloide neben der sich bildenden Emulsion einen zweiten Flüssigkeitsspeicher aus. Die Abgabe einer wundheilungsfördernden Substanz wird hierbei unter anderem durch die Aufnahme an Wundexsudat gesteuert. Je mehr Wundexsudat von der hydrophilen Basis oder den Hydrocolloiden aufgenommen wird, desto mehr an wundheilungsfördernder Substanz wird abgegeben. Die Hydrocolloide stellen somit einen Flüssigkeitsspeicher und ein Depot für eine wundheilungsfördernde Substanz dar, das eine wundheilungsfördernde Substanz kontrolliert freisetzt.

Die medizinische Zusammensetzung umfasst weniger als 10 Gew.-% Wasser, insbesondere weniger als 5 Gew.-% Wasser und ist ganz besonders bevorzugt wasserfrei. Insbesondere umfasst die hydrophile Basis weniger als 10 Gew.-% Wasser, insbesondere weniger als 5 Gew.-% Wasser und ist ganz besonders bevorzugt wasserfrei. Diese hydrophile Basis liegt somit als Einphasengemisch vor und ist durch den Anteil an mindestens einem Emulgator in der Lage, bei Zugabe von beispielsweise Wasser eine Emulsion auszubilden. Hiermit und im Folgenden ist im Zusammenhang mit der vorliegenden Erfindung gemeint, dass die hydrophile Basis Spuren an Wasser enthalten kann, wobei der Gehalt an Wasser dabei höchstens 1 Gew.-% bezogen auf die Masse der hydrophilen Basis betragen soll.

In einer bevorzugten Ausführungsform weist die Zusammensetzung eine hydrophile Basis auf, die eine Creme, Cremegrundlage oder Salbe ist. Insbesondere ist die hydrophile Basis eine hydrophile Creme oder Cremegrundlage oder eine hydrophile Salbe. Unter einer Salbe soll hierbei und im Kontext dieser Anmeldung ein Einphasensystem verstanden sein, wogegen eine Creme ein Zwei- oder Mehrphasensystem ist. Eine genaue Unterscheidung dieser Formulierungen auch in Abgrenzung von weiteren Formulierungen ist mit dem Deutschen Arzneibuch DAB 9 und dessen Kommentar gegeben auf das/ den hier ausdrücklich Bezug genommen wird.

Des Weiteren ist insbesondere vorgesehen, dass die Zusammensetzung eine hydrophile Basis umfasst, die weiterhin 10 bis 30 Gew.-% unpolare Lipide und ganz besonders bevorzugt weiterhin 10 bis 25 Gew.-% unpolare Lipide umfasst.

Im Rahmen der vorliegenden Erfindung wird als Oberbegriff für Fette, Öle, Wachse und dergleichen der Ausdruck "Lipide" verwendet. Auch werden die Begriffe "Ölphase" und "Lipidphase" synonym angewendet. Lipide unterscheiden sich unter anderem in ihrer Polarität. Es wurde bereits vorgeschlagen, die Grenzflächenspannung gegenüber Wasser als Maß für die Polarität eines Lipids bzw. einer Lipidphase anzunehmen. Dabei gilt, dass die Polarität der betreffenden Lipidphase umso größer ist, je niedriger die Grenzflächenspannung zwischen dieser Lipidphase und Wasser ist. Erfindungsgemäß wird die Grenzflächenspannung als ein mögliches Maß für die Polarität einer gegebenen Ölkomponente angesehen. Die Grenzflächenspannung ist dabei diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/ Länge und wird gewöhnlich in mN/ m wiedergegeben. Sie hat positives Vorzeichen, wenn sie das Bestreben hat, die Grenzfläche zu verkleinern. Im umgekehrten Falle hat sie negatives Vorzeichen. Als polar im Sinne der vorliegenden Erfindung werden insbesondere Lipide angesehen, deren Grenzflächenspannung gegen Wasser weniger als 20 mN/ m beträgt und als unpolar solche, deren Grenzflächenspannung gegen Wasser insbesondere mehr als 30 mN/ m beträgt. Lipide mit einer Grenzflächenspannung gegen Wasser zwischen 20 und 30 mN/ m werden im Allgemeinen als mittelpolar bezeichnet.

Unpolare Lipide sind insbesondere solche Lipide, welche gewählt werden aus der Gruppe der verzweigten und unverzweigten Kohlenwasserstoffe und Kohlenwasserstoffwachse, insbesondere Vaseline, Petrolatum, Paraffinöl, Mineralöl, Polyisobutene, Wax oder Kombinationen hiervon.

Neben unpolaren Lipidkomponenten kann die erfindungsgemäße hydrophile Basis auch polare und mittelpolare Lipide enthalten. Polare oder mittelpolare Lipide sind beispielsweise solche aus der Gruppe der Fettsäuretriglyceride, Fettsäurediglyceride, Fettsäuremonoglyceride oder Fettsäureester Oligomerer des Glycerins wie beispielsweise Voll- oder Partialfettsäureester des Diglycerins oder Triglycerins. Insbesondere können die Tri-, Di- und Monoglyceride Ester gesättigter und/oder ungesättigter, verzweigter und/oder unverzweigter Alkancarbonsäuren einer Kettenlänge von 8 bis 24, insbesondere 12 bis 18 C-Atomen sein. Die Fettsäuretriglyceride, Fettsäurediglyceride oder Fettsäuremonoglyceride können beispielsweise vorteilhaft gewählt werden aus der Gruppe der synthetischen, halbsynthetischen und natürlichen Fette oder Öle.

Als hydrophile Basis wird im Zusammenhang mit der vorliegenden Erfindung insbesondere auch ein Gemisch angesehen, das einen Anteil an polaren und unpolaren Lipiden und zu 0,5 - 40 Gew.-% mindestens einen Emulgator, insbesondere zu 0,5 - 30 Gew.-% mindestens einen Emulgator umfasst, wobei der Anteil an mittelpolaren und polaren Lipiden in der hydrophilen Basis im Verhältnis zu unpolaren Lipiden mehr als 1:1 insbesondere mehr als 2:1 und ganz besonders zwischen 3:1 und 10:1 bezogen auf den Gesamtgehalt an Lipiden beträgt.

Insbesondere weist eine erfindungsgemäße Zusammensetzung eine hydrophile Basis auf, die 20 - 80 Gew.-% Mono-, Di- und/ oder Triglyceride und/ oder Voll- oder Partialester Oligomerer des Glycerins bezogen auf das Gesamtgewicht der Zusammensetzung umfasst. Insbesondere umfasst die hydrophile Basis 30 - 70 Gew.-% und ganz besonders 40 - 70 Gew.-% Mono-, Di- und/ oder Triglyceride und/ oder Voll- oder Partialester Oligomerer des Glycerins bezogen auf das Gesamtgewicht der Zusammensetzung auf. Dabei ist es besonders vorteilhaft, wenn die Zusammensetzung 10 - 50 Gew.-% Mono-, Di- und/ oder Triglyceride und 10 - 30 Gew.-% Partialester Oligimerer des Glycerins insbesondere des Diglycerins oder der Triglycerins umfasst.

In einer weiteren besonders bevorzugten Ausführungsform weist eine erfindungsgemäße Zusammensetzung eine hydrophile Basis auf, die 40 - 80 Gew.-% Monoglyceride, Diglyceride, Triglyceride und/ oder Partialester Oligomerer des Glycerins insbesondere des Diglycerins oder des Triglycerins, 15 - 30 Gew.-% unpolare Fette und 0,5 - 30 Gew.-% Emulgator bezogen auf das Gesamtgewicht der Zusammensetzung umfasst.

In einer weiteren besonders bevorzugten Ausführungsform weist eine erfindungsgemäße Zusammensetzung eine hydrophile Basis auf, die 40 - 80 Gew.-% Monoglyceride, Diglyceride, Triglyceride und/ oder Partialester Oligomerer des Glycerins insbesondere des Diglycerins oder des Triglycerins, 15 - 30 Gew.-% unpolare Fette und 0,5 - 30 Gew.-% Emulgator umfasst, und 1 - 50 Gew.-% Hydrocolloide, die in der hydrophilen Basis dispergiert sind und die mindestens eine freisetzbare, wundheilungsfördernde Substanz umfassen.

Als Emulgatoren sollen im Zusammenhang mit der vorliegenden Erfindung solche Substanzen verstanden sein, die eine Grenzflächenaktivenaktivität aufweisen, so dass sich bei Zugabe von Wasser zu der hydrophilen Basis ein Mehrphasengemisch, nämlich eine Emulsion ausbilden kann. Insbesondere soll eine erfindungsgemäße Zusammensetzung mindestens einen Emulgator umfassen, durch den die hydrophile Basis in der Lage ist, bei Zugabe von Wasser eine Wasser-in-Öl-Emulsion (W/O-Emulsion), Gel-in-Öl-Emulsion (G/O-Emulsion), eine Öl-in-Wasser-Emulsion (O/W-Emulsion), Öl-in-Gel-Emulsion (O/G-Emulsion), Wasser-in-Öl-in-Wasser-Emulsion, (W/O/W-Emulsion) Gel-in-Öl-in-Gel-Emulsion (G/O/G-Emulsion), Gel-in-Öl-in Wasser-Emulsion (G/O/W-Emulsion), Wasser-in-ÖI-in-Gel-Emulsion (G/O/W-Emulsion), Öl-in-Wasser-in-Öl-Emulsion (O/W/O-Emulsion), oder Öl-in-Gel-in-Öl-Emulsion (O/G/O-Emulsion) auszubilden. Weiterhin bevorzugt sind solche Emulgatoren, die eine O/W- oder W/O-Emulsion oder eine O/G- oder G/O-Emulsion ausbilden können und frei von Ethylen- oder Propylenglykolen oder Ethylen-PropylenGlykolen sind, das heißt keinerlei Substanzen umfassen, die Ethylen-, Propylen- oder Ethylen-Propylen-Glykol-Einheiten umfassen.

Hierbei kann eine erfindungsgemäße Zusammensetzung insbesondere 0,5 - 50 Gew.-% mindestens eines Emulgators, insbesondere 0,5 - 40 Gew.-% mindestens eines Emulgators, insbesondere 0,5 - 30 Gew.-% mindestens eines Emulgators, insbesondere 1 - 20 Gew.-% mindestens eines Emulgators uns ganz besonders bevorzugt insbesondere 1 - 10 Gew.-% mindestens eines Emulgators aufweisen.

In einer weiteren Ausgestaltung der Erfindung umfasst damit eine erfindungsgemäße Zusammensetzung gemäß Anspruch 1 die mindestens eine freisetzbare wundheilungsfördernde Substanz umfassen, wobei die hydrophile Basis zu 0,5 bis 40 Gew.-% mindestens einen O/W-Emulgator, insbesondere einen ionischen O/W-Emulgator umfasst. Es kann jedoch auch vorgesehen sein, dass anstelle des O/W-Emulgators ein nichtionischer W/O-Emulgator eingesetzt wird. Diese Zusammensetzung weist weniger als 10 Gew.-% Wasser auf und umfasst weiterhin bevorzugt eine hydrophile Basis, die zu 0,5 bis 30 Gew.-% mindestens einen O/W-Emulgator, insbesondere einen ionischen O/W-Emulgator, oder einen W/O-Emulgator, insbesondere einen nichtionischen W/O-Emulgator, umfasst.

In einer weiteren Ausgestaltung der Erfindung umfasst damit eine erfindungsgemäße Zusammensetzung gemäß Anspruch 1, 60 bis 95 Gew.-% hydrophile Basis, in die 5 bis 40 Gew.-% Hydrocolloide dispergiert sind, wobei die hydrophile Basis 0,5 bis 50 Gew.-% mindestens einen O/W-Emulgator umfasst. Es kann jedoch auch vorgesehen sein, dass anstelle des O/W-Emulgator ein nichtionischer W/O-Emulgator eingesetzt wird.

Bei der Verwendung eines Emulgators vom O/W-Typ besteht der Vorteil darin, dass bei der Anwendung der Zusammensetzung die komplette Zusammensetzung besonders leicht mittels Wasser beispielsweise von einer Wunde abgewaschen werden kann.

Weiterhin bevorzugt kann eine erfindungsgemäße Zusammensetzung mindestens einen nichtionischen Emulgator mit einem HLB-Wert von 3 bis 18, gemäß den im Römpp-Lexikon Chemie (Hrg. J. Falbe, M. Regitz), 10. Auflage, Georg Thieme Verlag Stuttgart, New York, (1997), Seite 1764, aufgeführten Definitionen enthalten. Nichtionische O/W- Emulgatoren mit einem HLB-Wert von 10 bis 15 sowie nichtionische W/O-Emulgatoren mit einem HLB-Wert von 3 bis 6 sind erfindungsgemäß besonders bevorzugt.

Vorteilhaft kann oder können der oder die Emulgatoren insbesondere nichtionische O/W-Emulgatoren gewählt werden aus der Gruppe:
- der Fettalkoholethoxylate mit der allgemeinen Formel R-O-(CH₂-CH₂-O)ₙ-H oder der Fettalkoholpropoxylate mit der allgemeinen Formel R-O-(CH₂-CH(CH₃)-O)ₙ-H, wobei R einen verzweigten oder unverzweigten Alkyl- oder Alkenylrest und n eine Zahl von 10 bis 50 darstellen,
- der ethoxylierten oder propoxylierte Wollwachsalkohole,
- der Polyethylenglykolether mit der allgemeinen Formel R-O-(CH₂-CH₂-O)ₙ-R' oder der Polypropylenglykolether mit der allgemeinen Formel R-O-(CH₂-CH(CH₃)-O)ₙ-R', wobei R und R' unabhängig voneinander verzweigte oder unverzweigte Alkyl- oder Alkenylreste und n eine Zahl von 10 bis 80 darstellen,
- der Fettsäureethoxylate mit der allgemeinen Formel R-COO-(CH₂-CH₂-O)ₙ-H oder der Fettsäurepropoxylate mit der allgemeinen Formel R-COO-(CH₂-CH(CH₃)-O)ₙ-H, wobei R einen verzweigten oder unverzweigten Alkyl- oder Alkenylrest und n eine Zahl von 10 bis 40 darstellen,
- der veretherten Fettsäureethoxylate mit der allgemeinen Formel
   R-COO-(CH₂-CH₂-O)ₙ-R' oder der veresterten Fettsäurepropoxylate mit der allgemeinen Formel R-COO-(CH₂-CH(CH₃)-O)ₙ-R', wobei R und R' unabhängig voneinander verzweigte oder unverzweigte Alkyl- oder Alkenylreste und n eine Zahl von 10 bis 80 darstellen,
- der veresterten Fettsäureethoxylate mit der allgemeinen Formel
   R-COO-(CH₂-CH₂-O)ₙ-C(O)-R' oder der veresterten Fettsäurepropoxylate mit der allgemeinen Formel R-COO-(CH₂-CH(CH₃)-O)ₙ-C(O)-R', wobei R und R' unabhängig voneinander verzweigte oder unverzweigte Alkyl- oder Alkenylreste und n eine Zahl von 10 bis 80 darstellen,
- der Polyethylenglycolglycerinfettsäureester oder der Polypropylenglycolglycerinfettsäureester gesättigter und/oder ungesättigter, verzweigter und/oder unverzweigter Fettsäuren und einem Ethoxylierungsgrad oder Propoxylierungsgrad zwischen 3 und 50,
- der ethoxylierten oder propoxylierter Sorbitanester mit einem Ethoxylierungsgrad oder Propoxylierungsgrad von 3 bis 100,
- der ethoxylierten oder propoxylierte Triglyceride mit einem Ethoxylierungsgrad oder Propoxylierungsgrad zwischen 3 und 150,
- der Polyoxyethylensorbitolfettsäureester, basierend auf verzweigten oder unverzweigten Alkan- oder Alkensäuren und einen Ethoxylierungsgrad von 5 bis 100 aufweisend, beispielsweise vom Sorbeth- Typ.

Vorteilhaft im Rahmen der vorliegenden Erfindung einsetzbare Emulgatoren sind weiterhin nichtionische W/O-Emulgatoren aus der Gruppe der Dicarbonsäureester oder Tricarbonsäureester. Insbesondere sind hiervon Ester der Malonsäure, Bernsteinsäure, Adipinsäure geeignet. Weiterhin bevorzugt sind hiervon Ester der Dicarbonsäuren, insbesondere Ester der Bernsteinsäure geeignet, die mit gesättigten oder ungesättigten und/ oder linearen oder verzweigten C8-C24-Fettalkohole und/ oder Glycerin sowie deren Oligomeren, insbesondere Diglycerin oder Triglycerin gebildet werden. Insbesondere haben sich als nichtionische W/O-Emulgatoren Ester der Bernsteinsäure mit gesättigten und verzweigten C8-C24-Fettalkohole und/ oder Glycerin sowie deren Oligomeren, insbesondere Diglycerin oder Triglycerin als besonders vorteilhaft erwiesen. Ganz besonderes sind hiervon Dicarbonsäureester geeignet, die aus Bernsteinsäure und gesättigten und verzweigten C8-C24-Fettalkoholen und Diglycerin gebildet werden. Ein solcher Emulgator wird gemäß der INCI-Nomenklatur als Isostearyl Digyceryl Succinate bezeichnet und ist unter der Produktbezeichnung "Imwitor®780" erhältlich. Diese Emulgatoren haben den weiteren Vorteil, dass sie polyethylenglykol-frei sind, das heißt keine Einheiten von Ethylenglykol umfassen.

Als O/W-Emulgatoren können im Zusammenhang mit der vorliegenden Erfindung insbesondere auch ionische O/W-Emulgatoren verwendet werden. Als O/W-Emulgatoren insbesondere ionische O/W-Emulgatoren können vorteilhafterweise Emulgatoren ausgewählt werden aus der Gruppe der Ester von Monoglyceriden und/oder Diglyceriden gesättigter oder ungesättigter Fettsäuren mit Hydroxycarbonsäuren und/oder Tricarbonsäuren. Besonders bevorzugt sind als O/W-Emuigatoren partiell neutralisierte Ester von Monoglyceriden und/oder Diglyceriden gesättigter Fettsäuren mit Hydroxycarbonsäuren und/ oder Tricarbonsäuren, insbesondere der Milchsäure und/ oder der Zitronensäure. Ganz besonders bevorzugt sind Ester der Milchsäure und/ oder Zitronensäure die gemäß der INCI-Nomenklatur als Glyceryl Cocoate Citrate Lactate bezeichnet werden. Solche Emulgatoren sind beispielsweise unter der Produktbezeichnung "IMWITOR® 380" oder "IMWITOR® 377" erhältlich. Diese Emulgatoren haben den weiteren Vorteil, dass sie polyethylenglykol-frei sind, das heißt keine Einheiten von Ethylenglykol umfassen.

Gemäß der vorliegenden Erfindung soll unter einem Hydrocolloid ein Material verstanden sein, das ein hydrophiles synthetisches oder natürliches Polymermaterial ist, das löslich oder absorbierend und/ oder quellend in Wasser ist und ein Gel bildet. Vorzugsweise umfasst eine erfindungsgemäße Zusammensetzung ein Hydrocolloid aus einem synthetischen oder natürlichen Polymermaterial, das ausgewählt wird aus der Gruppe Alginsäure und deren Salze sowie deren Derivate, Chitin oder dessen Derivate, Chitosan oder dessen Derivate, Pektin, Cellulose oder dessen Derivate wie Celluloseether oder Celluloseester, vernetzte oder nicht vernetzte Carboxyalkylcellulose oder Hydroxyalkylcellulose, Polyvinylalkohol, Polyvinylpyrrolidon, Agar, Guargum oder Gelatine. Ganz besonders bevorzugt können als Hydrocolloid Cellulose oder Derivate oder deren Salze, Alginsäure oder deren Derivate oder Salze sowie Gemische hiervon verwendet werden.

Diese Hydrocolloide sind in der Lage eine wundheilungsfördernde Substanz aufzunehmen, diese Substanz als Depot zu speichern und bei Bedarf in Gegenwart von Flüssigkeiten wieder abzugeben. Überraschenderweise hat sich gezeigt, dass die Flüssigkeitsaufnahme und die Abgabe von Wirkstoffen auch in Gegenwart einer wässrigen Phase und einer Ölphase von statten geht. Hierbei hat sich insbesondere gezeigt, dass eine Abgabe von wundheilungsfördernder Substanz auch kontrolliert und über einen längeren Zeitraum stattfindet. Weiterhin überraschend hat sich gezeigt, dass durch die Gegenwart einer wässrigen Phase und einer Ölphase bei der Wahl der Wirkstoffe hinsichtlich ihrer Lipophilie oder Hydrophilie keine besonderen Grenzen gesetzt sind, d.h. durch die Art der sich bildenden Emulsionen kann entweder lipophile Wirksubstanz oder ein hydrophiler Wirkstoff vermehrt frei gesetzt werden.

Das Hydrocolloid kann sowohl in Form von Fasern als auch in Form von Partikeln und/ oder Fasern dispergiert in der Zusammensetzung vorliegen. Insbesondere kann das Hydrocolloid in Form von Partikeln vorliegen. Bevorzugt sind solche Hydrocolloide, die als Partikel vorliegen und die einen durchschnittlichen Partikeldurchmesser von 100 bis 1000 µm aufweisen. Der Anteil an gelbildenden Partikeln in der Zusammensetzung beträgt 1 bis 50 Gew.-% bezogen auf das Gesamtgewicht der Zusammensetzung. Vorzugsweise kann der Anteil an gelbildenden Partikeln 1 bis 40 Gew.-%, weiterhin vorzugsweise 1 bis 30 Gew.-% und ganz besonders bevorzugt 1 bis 25 Gew.-% bezogen auf das Gesamtgewicht der Zusammensetzung betragen. Der Anteil an Hydrocolloiden in der Zusammensetzung beträgt weiterhin bevorzugt 5 bis 40 Gew.-% bezogen auf das Gesamtgewicht der Zusammensetzung. Vorzugsweise kann der Anteil an Hydrocolloiden 5 bis 30 Gew.-%, weiterhin vorzugsweise 10 bis 25 Gew.-% und ganz besonders bevorzugt 15 bis 25 Gew.-% bezogen auf das Gesamtgewicht der Zusammensetzung betragen.

Besonders bevorzugt sind Hydrocolloide, die in Partikelform vorliegen, wobei die Partikel einen Wassergehalt von weniger als 10 Gew.-%, insbesondere weniger als 8 Gew.-%, insbesondere weniger als 5 Gew.-% bezogen auf die Hydrocolloidpartikel aufweisen. Ganz besonders bevorzugt sind Partikel, die wasserfrei sind. Hiermit und im Folgenden ist im Zusammenhang mit der vorliegenden Erfindung gemeint, dass die gelbildenden Partikel Spuren an Wasser enthalten können, wobei der Gehalt an Wasser dabei höchstens 1 Gew.-% bezogen auf die Masse der gelbildenden Partikel betragen soll.

Weiterhin bevorzugt können Hydrocolloide verwendet werden, die inter- und/ oder intramolekular vernetzt oder quervernetzt sind. Diese Hydrocolloide sind nicht löslich in beispielsweise Wasser oder Salzlösungen, das heißt diese Hydrocolloide quellen bei Zugabe dieser Flüssigkeiten und weisen einen inneren Zusammenhalt auf, dass die gequollenen Partikel dispers in der Zusammensetzung vorliegen.

Gemäß einer besonders bevorzugten Ausführungsform umfasst eine erfindungsgemäße Zusammensetzung vorzugsweise mindestens ein Hydrocolloid ausgewählt aus der Gruppe der Cellulose-Derivate oder deren Salze, Alginate oder deren Derivate, Chitin oder dessen Derivate oder dessen Salze. Hierbei ist der Ursprung der Hydrocolloide unbeachtlich, das heißt, dass diese Hydrocolloide pflanzlichen oder tierischen Ursprungs sein können oder auf synthetischem Weg beispielsweise durch mikrobiologische Verfahren hergestellt sein können. Es ist auch möglich, Hydrocolloide zu verwenden, die pflanzlichen oder tierischen Ursprungs und durch chemische Synthese modifiziert sind.

Zu der Gruppe der Cellulose-Derivate zählen im Zusammenhang mit der vorliegenden Erfindung insbesondere Celluloseether und Celluloseester sowie deren Salze. Als Celluloseether kommen hierbei insbesondere Hydroxyalkylcellulosen insbesondere Hydroxy-C₁₋₆-alkylcellulose wie zum Beispiel Hydroxymethylcellulose, Hydroxyethylcellulose, Hydroxypropylcellulose oder Hydroxybutylcellulose und ganz besonders bevorzugt Hydroxymethylcellulose oder Hydroxyethylcellulose zum Einsatz. Als Celluloseester kommen insbesondere Carboxyalkylcellulose insbesondere Carboxy-C₁₋₆-alkylcellulose wie zum Beispiel Carboxymethylcellulose, Carboxyethylcellulose, Carboxypropylcellulose oder Carboxybutylcellulose odere deren Salze und ganz besonders bevorzugt Carboxymethylcellulose oder Carboxyethylcellulose oder deren Salze zum Einsatz.

Gemäß einer weiteren bevorzugten Ausführungsform umfasst die Zusammensetzung mindestens zwei verschiedene Hydrocolloide. Dabei hat es sich als besonders vorteilhaft erwiesen, wenn die mindestens zwei Hydrocolloide ausgewählt werden aus der Gruppe der Cellulose-Derivate oder deren Salze insbesondere Celluloseester oder deren Salze, Alginate oder deren Derivate, Chitin oder dessen Derivate oder dessen Salze.

Weiterhin kann als Hydrocolloid auch ein synthetisches Polymermaterial eingesetzt werden, das aus der Gruppe der Polyacrylate oder deren Derivate oder Salze gewählt wird. Hierbei wird im Rahmen der vorliegenden Erfindung unter einem Polyacrylat ein synthetisches Polymer umfassend als Monomer (M1) Acrylsäure (2-Propensäure, CH2=CH-CO₂H) und/ oder ein Salz hiervon verstanden, das einen Monomeranteil von mehr als 60 Gew.-% Acrylsäure und/oder eines Salzes hiervon (bezogen auf das Gesamtgewicht des Polyacrylats) aufweist. Insbesondere weisen erfindungsgemäße Polyacrylate einen Monomeranteil von mehr als 80 Gew.-% Acrylsäure und/oder eines Salzes hiervon und ganz besonders bevorzugt mehr als 95 Gew.-% Acrylsäure und/oder eines Salzes hiervon bezogen auf das Gesamtgewicht des Polyacrylats auf. Dabei kann das Polyacrylat als Homopolymer, Copolymer oder Blockpolymer vorliegen. Falls das Polyacrylat als Copolymer oder Blockpolymer vorliegt, so liegt in jedem Fall der Monomeranteil des Monomers M1 in dem Polymer bei mehr als 60 %, insbesondere bei mehr als 80 % und ganz besonders bevorzugt bei mehr als 95 % bezogen auf das Gesamtgewicht des Polyacrylats. In diesen copolymeren Polyacrylaten oder blockpolymeren Polyacrylaten können neben dem Monomer M1 als Comonomere M2 insbesondere α,β-ungesättigte Ether (Vinylether), α,β-ungesättigte Carbonsäuren oder α,β-ungesättigte Carbonsäureester (Vinylester) enthalten sein. Von den Comonomeren M2 der α,β-ungesättigten Carbonsäuren werden besonders Methacrylsäure (2-Methylpropensäure), Ethacrylsäure (2-Ethylpropensäure), Crotonsäure (2-Butensäure), Sorbinsäure (trans-trans-2,4-Hexadiensäure), Maleinsäure (cis-2-Butendisäure) oder Fumarsäure (trans-2-Butendisäure) bevorzugt. In einer besonders bevorzugten Form der Erfindung kann jedoch auch vorgesehen sein, dass das Polyacrylat aus a) einem Homopolymer aus Acrylsäure und/oder b) einem Copolymer aus i) Acrylsäure und einem Salz der Acrylsäure, ii) aus Methacrylsäure und einem Salz der Methacrylsäure oder iii) aus Acrylsäure und Methacrylsäure und deren Salze besteht. Weiterhin kann jedoch auch vorgesehen sein, dass es sich bei dem Polyacrylat um eine Mischung von verschiedenen Polyacrylaten handelt.

Hierbei können insbesondere die α,β-ungesättigten Carbonsäuren als auch die Acrylsäure in neutralisierter Form als Salz, in nicht-neutralisierter Form als freie Säure oder in Mischungen hiervon vorliegen. Insbesondere haben sich Polyacrylate, die aus Acrylsäure und Salzen der Acrylsäure aufgebaut sind als besonders wirksam gezeigt. Dabei sind insbesondere Alkalimetall- oder Erdalkalimetallsalze hervorzuheben. Insbesondere zeigten sich Polyacrylate, die aus Homopolymeren und/oder aus Copolymeren bestehen, die als Monomere Acrylsäure und/ oder Natrium- oder Kaliumacrylat umfassen, als besonders wirksam.

Weiterhin hat sich in überraschender Weise gezeigt, dass die vorliegende Aufgabe in besonderes hervorzuhebender Weise durch Hydrocolloide aus der Gruppe der vernetzen und/ oder quervernetzten Polyacrylate gelöst wird. Diese Polyacrylate umfassen vorzugsweise a) ein Homopolymer, das aus den Monomeren M1 besteht und mittels eines Vernetzers vernetzt und/ oder quervernetzt ist, und/ oder b) ein Copolymer, das aus den Monomeren M1 und M3 besteht, wobei das Monomer M1 Acrylsäure und/ oder ein Salz hiervon ist und das Monomer M3 aus der Gruppe der Vernetzer gewählt wird. Das heißt, dass diese Polyacrylate ein mittels eines Vernetzers nachträglich vernetztes Polyacrylat und/ oder ein Polyacrylat umfassen, das aus Acrylsäure und/ oder einem Salz hiervon und einem Vernetzer copolymerisiert ist.

Insbesondere hat sich gezeigt, dass vernetze und/ oder quervernetzte Polyacrylate, die als Vernetzer Verbindungen V1 enthalten, die mindestens zwei ethylenisch ungesättigte Gruppen innerhalb eines Moleküls aufweisen, oder Verbindungen V2, die mindestens zwei funktionelle Gruppen aufweisen, die mit funktionellen Gruppen der Acrylsäure oder/ und eines Salzes hiervon in einer Kondensationsreaktion, in einer Additionsreaktion oder in einer Ringöffnungsreaktion reagieren können, oder Verbindungen V3, die mindestens eine ethylenisch ungesättigte Gruppe und mindestens eine funktionelle Gruppe, die mit funktionellen Gruppen der Acrylsäure und/ oder eines seiner Salze und/ oder den α,β-ungesättigte Comonomere in einer Kondensationsreaktion, in einer Additionsreaktion oder in einer Ringöffnungsreaktion reagieren kann, besonders wirksam sind. Dabei wird durch die Verbindungen V1 eine Vernetzung der Polymere durch die radikalische Polymerisation der ethylenisch ungesättigten Gruppen des Vernetzermoleküls mit den monoethylenisch ungesättigten Monomeren Acrylsäure und/ oder eines seiner Salze und/ oder einer der α,β-ungesättigte Comonomere erreicht, während bei den Verbindungen V2 eine Vernetzung der Polymere durch Kondensationsreaktion der funktionellen Gruppen mit den funktionellen Gruppen der Acrylsäure und/ oder eines seiner Salze oder einer α,β-ungesättigte der Comonomere erreicht wird. Bei den Verbindungen V3 erfolgt dementsprechend eine Vernetzung des Polymers sowohl durch radikalische Polymerisation der ethylenisch ungesättigten Gruppe als auch durch Kondensationsreaktion zwischen der funktionellen Gruppe des Vernetzers und den funktionellen Gruppen der Monomeren.

Bevorzugte Verbindungen V1 sind Polyacrylsäureester oder Polymethacrylsäureester, die beispielsweise durch die Umsetzung eines Polyols, wie beispielsweise Ethylenglykol (1,2-Ethandiol), Propylenglykol (1,2-Propandiol), Trimethylolpropan (2-Ethyl-2-hydroxymethyl-1,3-propandiol), 1,6-Hexandiol, Glycerin (1,2,3-Propantriol), Pentaerythrit (2,2-Bis(hydroxymethyl)propan-1,3-diol), Polyethylenglykol (HO-(CH₂-CH₂-O)ₙ-H mit n = 2 bis 20) mit n = 1 bis 20), Polypropylenglykol (HO-(CH(CH₃)-CH₂-O)ₙ-H mit n = 2 bis 20), eines Aminoalkohols, eines Polyalkylenpolyaminens, wie beispielsweise Diethylentriamin oder Triethylentetraamin, oder eines alkoxylierten Polyols mit Acrylsäure oder Methacrylsäure gewonnen werden. Besonders bevorzugt kann es sich bei dem vernetzten Polyacrylaten um ein Polyacrylat handeln, das mittels einer Verbindung V1 vernetzt ist, die ein Di-, Tri- oder Tetraester der Polyacrylsäure oder Polymethacrylsäure ist, der durch die Umsetzung eines alkoxylierten Polyols, insbesondere eines ethoxylierten Polyols, insbesondere ethoxyliertes Ethylenglycol, ethoxyliertes Propylenglycol, ethoxyliertes Trimethylolpropan, ethoxyliertes 1,6-Hexandiol oder ethoxyliertes Glycerin, mit einer durchschnittlichen Anzahl an Ethylenoxideinheiten n pro Hydroxygruppe von n = 1 bis 10 mit Acrylsäure oder Methacrylsäure gewonnen werden. Als Verbindungen V1 sind des Weiteren Polyvinylverbindungen, Polyallylverbindungen, Polymethylallylverbindungeh, Acrylsäureester oder Methacrylsäureester einer Monovinylverbindung, Acrylsäureester oder Methacrylsäureester einer Monoallylverbindung oder Monomethylallylverbindung, vorzugsweise der Monoallylverbindungen oder Monomethylallylverbindungen eines Polyols oder eines Aminoalkohols, bevorzugt. In diesem Zusammenhang wird auf DE 195 43 366, DE 195 43 368 verwiesen.

Überraschenderweise wird die oben aufgeführte Aufgabe auch durch Hydrocolloide aus der Gruppe der Polyacrylat-Superabsorber gelöst. Hierbei wird im Rahmen der vorliegenden Erfindung unter einem Polyacrylat-Superabsorber ein Polyacrylat mit den oben aufgeführten Merkmalen verstanden, das in der Lage ist, mindestens das 15-fache seines Eigengewichtes an physiologischer Kochsalzlösung (0,9 %-ige NaCl-Lösung, Wasser) zu absorbieren.

Superabsorber sind in der modernen Wundbehandlung lange Zeit bekannt. Diese werden jedoch lediglich als Wundflüssigkeit aufsaugendes Medium verwendet. Werden diese Superabsorber in dieser Art und Weise eingesetzt, wird einzig und allein der Flüssigkeitshaushalt der Wunde oder des Wundbetts reguliert. Überraschenderweise hat sich nun auch gezeigt, das Polyacrylat-Superabsorber in der Lage sind, in Gegenwart einer Wasserphase und einer Ölphase sowohl Flüssigkeiten aufzunehmen als auch eine wundheilungsfördernde Substanz abzugeben. Auch diese Abgabe erfolgt kontrolliert über einen längeren Zeitraum.

In jedem Fall kann der Polyacrylat-Superabsorber in Form von Fasern oder Partikeln oder Gelen vorliegen. Dabei kann der Polyacrylat-Superabsorber in trockener oder in bereits gequollener Form vorliegen. In gequollener Form liegt das Polymer in gelförmigen, diskreten Fasern oder Partikeln vor. Dabei kann ein erfindungsgemäßes Hydrocolloid aus der Gruppe der Polyacrylat-Superabsorber in Form von Partikeln vorliegen, die mit Wasser, physiologischer Kochsalz-Lösung oder Ringer-Lösung versetzt sind, wobei die Partikel bereits in gequollener Form vorliegen.

Dabei zeigten sich zudem Polyacrylat-Superabsorber und insbesondere vernetzte Polyacrylat-Superabsorber als besonders bevorzugt, die als Partikel vorliegen und eine Partikelgrößenverteilung aufweisen, deren Anteil an Partikel zwischen 850 µm und 300 µm mindestens 55 %, insbesondere mindestens 65 % und ganz besonders mindestens 70 % beträgt (gemessen nach EDANA 420.2-02). Insbesondere sind auch superabsorbierende Polyacrylate bevorzugt, deren Partikelanteil an Partikeln kleiner als 850 µm mindestens 85 %, besonders mindestens 90 % und ganz besonders mindestens 95 % bezogen auf alle Partikel beträgt (gemessen nach EDANA 420.2-02).

Als erfindungsgemäße aus einem Hydrocolloid freisetzbare wundheilungsfördernde Substanz umfasst eine erfindungsgemäße Zusammensetzung oder eine erfindungsgemäße Wundkontaktschicht insbesondere mindestens eine Substanz ausgewählt aus der Gruppe der Vitamine oder Provitamine, Carotinoide, Analgetika, Antiseptika, Hämostyptika, Antihistaminika, antimikrobiellen Metalle oder deren Salze, pflanzlichen wundheilungsfördernden Substanzen oder Substanzgemische, Pflanzenextrakte, Enzyme, Wachstumsfaktoren, Enzyminhibitoren sowie Kombinationen hiervon.

Als erfindungsgemäße Analgetika sind insbesondere nicht-steroidale Analgetika insbesondere Salicylsäure, Acetylsalicylsäure und deren Derivate z.B. Aspirin®, Anilin und dessen Derivate, Acetaminophen z.B. Paracetamol®, Antranilsäure und deren Derivate z.B. Mefenaminsäure, Pyrazol oder dessen Derivate z.B. Methamizol, Novalgin®, Phenazon, Antipyrin ®, Isopropylphenazon und ganz besonders bevorzugt Arylessigsäuren sowie deren Derivate, Heteroarylessigsäuren sowie deren Derivate, Arylpropionsäuren sowie deren Derivate und Herteroarylpropionsäuren sowie deren Derivate z.B. Indometacin®, Diclophenac®, Ibuprofen®, Naxoprophen®, Indomethacin®, Ketoprofen®, Piroxicam® geeignet.

Als erfindungsgemäße Wachstumsfaktoren sind insbesondere zu nennen: aFGF (Acidic Fibroplast Growth Factor), EGF (Epidermal) Growth Factor), PDGF (Platelet Derived Growth Factor), rhPDGF-BB (Becaplermin), PDECGF (Platelet Derived Endothelial Cell Growth Factor), bFGF (Basic Fibroplast Growth Factor),TGF α (Transforming Growth Factor alpha), TGF β (Transforming Growth Factor beta), KGF (Keratinocyte Growth Factor), IGF1/IGF2 (Insulin-Like Growth Factor) und TNF (Tumor Necrosis Factor).

Als erfindungsgemäße Vitamine oder Provitamine sind insbesondere die fettlöslichen oder wasserlöslichen Vitamine Vitamin A, Gruppe der Retinoide, Provitamin A, Gruppe der Carotenoide, insbesondere β-Carotin, Vitamin E, Gruppe der Tocopherole, insbesondere α-Tocopherol, β-Tocopherol, γ-Tocopherol, δ-Tocopherol und α-Tocotrienol, β-Tocotrienol, γ-Tocotrienol und δ-Tocotrienol, Vitamin K, Phyllochinon insbesondere Phytomenadion oder pflanzliches Vitamin K, Vitamin C, L-Ascorbinsäure, Vitamin B1, Thiamin, Vitamin B2, Riboflavin, Vitamin G, Vitamin B3, Niacin, Nikotinsäure und Nikotinsäureamid, Vitamin B5, Pantothensäure, Provitamin B5, Panthenol oder Dexpanthenol, Vitamin B6, Vitamin B7, Vitamin H, Biotin, Vitamin B9, Folsäure sowie Kombinationen hiervon geeignet.

Als Antiseptikum ist erfindungsgemäß solches Mittel zu verwenden, das gemizid, bakterizid, bakteriostatisch, fungizid, viruzid, virustatisch und/ oder allgemein mikrobiozid wirkt. Insbesondere sind gemäß der vorliegenden Erfindung solche Stoffe geeignet die ausgewählt werden aus der Gruppe Resorcinol, lod, lod-Povidon, Chlorhexidin, Benzalkoniumchlorid, Benzoesäure, Benzoylperoxid oder Cethylpyridiniumchlorid. Darüber hinaus sind als Antiseptika insbesondere auch antimikrobiellen Metalle zu verwenden. Als antimikrobielle Metalle können insbesondere Silber, Kupfer oder Zink sowie deren Salze, Oxide oder Komplexe in Kombination oder alleine verwendet werden.

Als pflanzliche wundheilungsfördernden Substanzen oder Substanzgemische oder Pflanzenextrakte sind im Zusammenhang mit der vorliegenden Erfindung insbesondere Extrakte der Kamille, Hamamelis-Extrakte z.B. Hamamelis virgina, Calendula-Extrakt, Aloe-Extrakt z.B. Aloe vera, Aloe barbadensis, Aloe feroxoder oder Aloe vulgaris, Grüntee-Extrakte, Meeresalgen-Extrakt z.B. Rotalgen- oder Grünalgen-Extrakt, Avocado-Extrakt, Myrre-Extrakt z.B. Commophora molmol, Bambus-Extrakte sowie Kombinationen hiervon zu verwenden. Hierbei sind erfindungsgemäß besonders Extrakte der Blätter, Blüten, Stengel oder Wurzeln der Pflanzen oder Kombinationen hiervon zu verstehen.

Die wundheilungsfördernden Substanzen, die einzeln oder als Gemisch verschiedener wundheilungsfördernder Substanzen eingesetzt werden können, sind insbesondere zu 0,01 bis 30 Gew.-%, bevorzugt zu 0,01 bis 15 Gew.% und ganz besonders bevorzugt zu 0,1 bis 10 Gew.-% bezogen auf das Gewicht der Hydrocolloide in der Zusammensetzung enthalten.

Damit umfasst eine erfindungsgemäße Zusammensetzung gemäß Anspruch 1, die zu 0,5 bis 40 Gew.-% mindestens einen O/W-Emulgator, insbesondere einen ionischen O/W-Emulgator, oder einen W/O-Emulgator, insbesondere ein nichtionischen W/O-Emulgator, umfasst. In der hydrophilen Basis sind 1 bis 50 Gew.-% Hydrokolloide dispergiert, die mindestens eine freisetzbare wundheilungsfördernde Substanz umfassen, wobei die wundheilungsfördernde Substanz ausgewählt wird aus der Gruppe der Vitamine oder Provitamine, Carotinoide, Analgetika, Antiseptika, Hämostyptika, Antihistaminika, antimikrobiellen Metalle oder deren Salze, pflanzlichen wundheilungsfördernden Substanzen oder Substanzgemische, Pflanzenextrakte, Enzyme, Wachstumsfaktoren, Enzyminhibitoren sowie Kombinationen hiervon.

Weiterhin ist auch eine Wundsalbe umfassend eine medizinische Zusammensetzung der oben beschriebenen Art Gegenstand der vorliegenden Erfindung.

Diese Wundsalbe kann besonders bei mäßig bis stark nässenden Wunden zur Anwendung gebracht werden, da mit dieser Wundsalbe einerseits in besonderem Maß Wundexsudat aufgesaugt wird und andererseits durch den Anteil an Hydrocolloiden, die einen Speicher für Flüssigkeiten bilden, ein feuchtes Klima einer Wunde bereitgestellt wird. Die Hydrocolloide wirken in der Wundsalbe zugleich als Flüssigkeitsspeicher und Feuchtigkeitsspender als auch als Wirkstoffdepot für wundheilungsfördernde Substanzen.

Diese Wundsalbe kann besonders zur Anwendung bei mäßig bis stark nässenden Wunden zur Anwendung gebracht werden, da mit dieser Wundsalbe einerseits in besonderem Maß Wundexsudat aufgesaugt wird und andererseits durch den Anteil an Hydrocolloiden, die einen Speicher für Flüssigkeiten bilden, ein feuchtes Klima einer Wunde bereitgestellt wird. Die Hydrocolloide wirken in der Wundsalbe zugleich als Flüssigkeitsspeicher als auch als Feuchtigkeitsspender.

Besonders bevorzugt weist die Wundsalbe gemäß Anspruch 1 weniger als 10 Gew.-% Wasser auf und umfasst weiterhin 50 bis 99 Gew.-% hydrophile Basis, in die 1 bis 50 Gew.-% Hydrokolloide dispergiert sind, die mindestens einen freisetzbaren, wundheilungsfördernde Substanz umfassen, wobei die hydrophile Basis 10 - 40 Gew.-% unpolare Fette und zu 0,5 - 40 Gew.-% mindestens einen Emulgator umfasst. Ganz besonders bevorzugt umfasst die erfindungsgemäße Wundsalbe mit weniger als 10 Gew.-% Wasser weiterhin zu 50 bis 99 Gew.-% eine hydrophile Basis, in die 1 bis 50 Gew.-% Hydrokolloide dispergiert sind, die mindestens eine freisetzbare, wundheilungsfördernde Substanz umfassen, wobei die hydrophile Basis 50 - 80 Gew.-% Monoglyceride, Diglyceride, Triglyceride und/ oder Partialester Oligomerer des Glycerins insbesondere des Diglycerins oder des Triglycerins, 20 - 35 Gew.-% unpolare Fette und 0,5 - 30 Gew.-% mindestens eines Emulgators umfasst. Insbesondere ist die Wundsalbe wasserfrei.

Besonders bevorzugt weist die Wundsalbe gemäß Anspruch 1 weniger als 10 Gew.-% Wasser auf und umfasst weiterhin 60 bis 95 Gew.-% hydrophile Basis, in die 5 bis 40 Gew.-% Hydrocolloide dispergiert sind, wobei die hydrophile Basis 20 - 35 Gew.-% unpolare Fette und zu 0,5 - 30 Gew.-% mindestens einen Emulgator umfasst. Ganz besonders bevorzugt umfasst die erfindungsgemäße Wundsalbe mit weniger als 10 Gew.-% Wasser weiterhin zu 60 bis 95 Gew.-% eine hydrophile Basis, in die 5 bis 40 Gew.-% Hydrocolloide dispergiert sind, wobei die hydrophile Basis 50 - 80 Gew.-% Monoglyceride, Diglyceride, Triglyceride und/ oder Partialester Oligomerer des Glycerins insbesondere des Diglycerins oder des Triglycerins, 20 - 35 Gew.-% unpolare Fette und 0,5 - 30 Gew.-% mindestens eines Emulgators umfasst. Insbesondere ist die Wundsalbe wasserfrei.

Damit ist auch die Verwendung einer Zusammensetzung gemäß Anspruch 15 mit weniger als 10 Gew.-% Wasser umfassend 60 bis 95 Gew.-% einer hydrophilen Basis, in die 5 bis 40 Gew.-% Hydrocolloide dispergiert sind, wobei die hydrophile Basis 0,5 bis 50 Gew.-% an mindestens einem Emulgator umfasst, zur Herstellung eines Mittels zur Behandlung von Wunden Gegenstand der vorliegenden Erfindung. Insbesondere kann das Mittel eine Wundsalbe sein, die weiterhin bevorzugt zur Behandlung von Brandwunden oder chronischen Wunden eingesetzt wird.

Weiterhin ist auch die Verwendung einer Zusammensetzung gemäß Anspruch 15 mit insbesondere weniger als 10 Gew.-% Wasser umfassend 50 bis 99 Gew.-% einer hydrophilen Basis, in die 1 bis 50 Gew.-% Hydrokolloide dispergiert, die mindestens eine freisetzbare, wundheilungsfördernde Substanz umfasst, wobei die hydrophile Basis 0,5 bis 40 Gew.-% an mindestens einem Emulgator umfasst, zur Herstellung einer Wundsalbe zur Behandlung von Wunden insbesondere zur Behandlung von Brandwunden oder chronischen Wunden Gegenstand der vorliegenden Erfindung.

Gemäß einem weiterführenden Gedanken der vorliegenden Erfindung ist auch eine Wundkontaktschicht umfassend ein Trägermaterial und eine medizinische Zusammensetzung Gegenstand der vorliegenden Erfindung. Insbesondere ist eine Wundkontaktschicht umfassend ein Trägermaterial für eine Zusammensetzung gemäß Anspruch 1, Gegenstand der vorliegenden Erfindung. Insbesondere umfasst die Zusammensetzung weniger als 10 Gew.-% Wasser, insbesondere weniger als 5 Gew.-% Wasser und ist ganz besonders bevorzugt wasserfrei.

Durch das Trägermaterial wird erreicht, dass die Wundkontaktschicht eine leicht applizierbare Form erhält, die direkt flächig auf eine Wunde aufgebracht werden kann. Dabei kann vorgesehen sein, dass die Zusammensetzung auf mindestens eine Seite des Trägermaterials gestrichen oder in sonstiger Art und Weise aufgebracht ist. Es kann auch vorgesehen sein, dass die Zusammensetzung auf beiden Seiten auf das Trägermaterial aufgebracht ist oder dass das Trägermaterial vollständig mit der Zusammensetzung imprägniert ist. Hierbei kann eine erfindungsgemäße Wundkontaktschicht weiterhin alle bevorzugten Eigenschaften oder Merkmale der oben beschriebenen Zusammensetzung aufweisen.

Ein weiterer Vorteil gegenüber bekannten Wundkontaktschichten besteht darin, dass bei der Applikation auf eine Wunde, die normalerweise von geschultem Personal mittels Handschuhen durchgeführt wird, eine erfindungsgemäße Wundkontaktschicht nicht an den Handschuhen haftet oder klebt. Somit sind diese Wundkontaktschichten besonders handhabungssicher.

Besonders vorteilhaft ist ein Auftrag einer Zusammensetzung insbesondere einer Salbe, Creme oder Cremegrundlage auf dem Trägermaterial in einer Menge von mindestens 50 g/ m², insbesondere von mindestens 100 g/ m² und besonders bevorzugt von 100 bis 450 g/ m² und ganz besonders bevorzugt von 100 bis 300 g/ m² vorzusehen.

Besonders bevorzugt umfasst die erfindungsgemäße Wundkontaktschicht ein Trägermaterial und eine Zusammensetzung gemäß Anspruch 1 mit weniger als 10 Gew.-% Wasser, wobei die Zusammensetzung einer 60 bis 95 Gew.-% hydrophilen Basis in die 5 bis 40 Gew.-% Hydrocolloide dispergiert sind, umfasst und wobei die hydrophile Basis 50 - 80 Gew.-% Monoglyceride, Diglyceride, Triglyceride und/ oder Partialester Oligomerer des Glycerins insbesondere des Diglycerins oder des Triglycerins, 20 - 35 Gew.-% unpolare Fette und 0,5 - 30 Gew.-% mindestens eines Emulgators umfasst. Insbesondere ist die Zusammensetzung wasserfrei.

Durch den Anteil an Fettsäureglyceriden in der Zusammensetzung wird der eine Wunde umgebenden Haut, der sogenannten peripheren Wundhaut, pflegende Bestandteile bereitgestellt, die eine Wundheilung in besonderem Maße unterstützen.

Als Trägermaterial können hierbei verschiedenste Materialien eingesetzt werden. Insbesondere hat sich herausgestellt, dass hierzu Polymerfilme oder-folien, Polymerschäume, Nonwoven sowie textile Materialien eingesetzt werden können. Als Trägermaterial in einer erfindungsgemäßen Wundkontaktschicht können insbesondere Nonwoven sowie textile Materialien wie Gestricke, Gewirke oder Gewebe eingesetzt werden. Ganz besonders bevorzugt können hierbei hydrophobe Gestricke, Gewirke oder Gewebe, die selbst keine Flüssigkeiten absorbieren verwendet werden. Insbesondere umfasst eine erfindungsgemäße WunoKoniaKtschicht als Trägermaterial ein Polyamid-Gewirk.

Wird ein textiles Trägermaterial eingesetzt, so kann dass Material insbesondere auch mit Öffnungen versehen sein, das heißt, dass das Trägermaterial mit Löchern versehen ist oder in Gitterform vorliegt. Insbesondere ist vorgesehen, dass das Trägermaterial ein Gewirk, Gewebe oder Gestrick ist, das Löcher aufweist, deren maximale lichte Weite im Bereich von 0,3 bis 3,0 mm vorzugsweise von 0,5 bis 2,5 mm und besonders bevorzugt von 0,5 bis 2,0 mm im ungedehnten Zustand des Materials beträgt. Hierbei können die Löcher jede beliebige Form annehmen wie z.B. kreisförmig, elliptisch, quadratisch, sechseckig oder achteckig. Diese Gewirke weisen ein Flächengewicht von mindestens 20 g/ m² bis höchstens 120 g/ m² auf.

Weiterhin kann vorgesehen sein, durch die Wundkontaktschicht mindestens eine die Wundheilung fördernde Substanz freizusetzen. Hierzu zählen insbesondere Substanzen, die fungizid, bakterizid oder antimikrobiell wirken. In einer besonderen Ausführungsform umfasst die Wundkontaktschicht ein Hydrocolloid, das seinerseits mindestens ein fungizid, bakterizid oder antimikrobiell Substanz umfasst. Ganz besonders sind hierbei Chitosan, Silber, Silberkomplexe, Silbersalze, Zink, Zinksalze oder Zinkkomplexe geeignet.

Besonders bevorzugt weist damit die Wundkontaktschicht ein Trägermaterial und eine medizinische Zusammensetzung gemäß Anspruch 9 mit weniger als 10 Gew.-% Wasser auf, wobei die Zusammensetzung zu 60 bis 95 Gew.-% hydrophile Basis umfasst, in die zu 5 bis 40 Gew.-% mindestens eine freisetzbare wundheilungsfördernde Substanz umfassende Hydrocolloide dispergiert sind, und wobei die hydrophile Basis 20 - 35 Gew.-% unpolare Fette und 0,5 - 30 Gew.-% mindestens eines Emulgators umfasst. Ganz besonders bevorzugt umfasst die erfindungsgemäße Wundkontaktschicht ein Trägermaterial und eine Zusammensetzung gemäß Anspruch 9 mit weniger als 10 Gew.-% Wasser, wobei die Zusammensetzung zu 60 bis 95 Gew.-% eine hydrophile Basis, in die 5 bis 40 Gew.-% Hydrokolloide dispergiert sind, umfasst und wobei die hydrophile Basis 50 - 80 Gew.-% Monoglyceride, Diglyceride, Triglyceride und/ oder Partialester Oligomerer des Glycerins insbesondere des Diglycerins oder des Triglycerins, 20 - 35 Gew.-% unpolare Fette und 0,5 - 30 Gew.-% mindestens eines Emulgators umfasst. Insbesondere ist die Zusammensetzung wasserfrei.

Es kann jedoch auch vorgesehen sein, dass ein wundheilungsförderndes Mittel direkt auf das Trägermaterial aufgebracht ist. In besonders vorteilhafter Weise wird in einer weiteren Ausführung der Wundkontaktschicht als Trägermaterial ein Nonwoven oder ein textiles Material wie Gestrick, Gewirk oder Gewebe verwendet, das mit einem antimikrobiell wirkenden Metall, vorzugsweise Silber oder Silbersalzen, beschichtet ist. Bei der Verwendung eines solchen Trägermaterials kann die Zusammensetzung direkt auf eine erste Seite des Trägermaterials auf das Metall oder Metallsalz aufgebracht werden. Dabei ist es besonders vorteilhaft, wenn die Zusammensetzung wasserfrei ist.

Dass das Trägermaterial mindestens auf seiner ersten Seite eine Zusammensetzung umfasst, ist dabei erfindungsgemäß so zu verstehen, dass die Zusammensetzung entweder unmittelbar auf dem mit Metall versehenen Träger angeordnet ist oder zunächst auf der ersten Seite eine kontinuierliche oder diskontinuierliche Metallschicht angebracht ist, auf die dann wiederum die Zusammensetzung aufgebracht ist. Es kann auch ein beidseitiges Aufbringen der Zusammensetzung erwünscht sein, insbesondere wenn ein Eintamponieren der Wundauflage vorgenommen werden soll. In diesem Fall ist es vorteilhaft, wenn auch der Metallauftrag beidseitig oder das Gewirk umschließend aufgebracht ist.

Dabei wirkt die Zusammensetzung, die insbesondere eine Salbe oder Creme sein kann, als Vermittler zwischen dem mit dem Metall ausgerüsteten Trägermaterial und der Wunde des Patienten. Auf diese Weise kann ein unmittelbarer Kontakt der Wunde mit dem Metall und insbesondere auch ein Verkleben desselben sicher vermieden werden. Darüber hinaus kann diese Salbe oder Creme im Bereich der peripheren Wundhaut eine pflegende Wirkung erzeugen. Tritt die Wundkontaktschicht dann über die Zusammensetzung mit der Wunde in Kontakt, wird insbesondere über die Wundflüssigkeit durch Vermittlung der Zusammensetzung das Metall, z. B. Silber aus der Wundauflage freigesetzt und gelangt über die Zusammensetzung in die Wunde. Dabei kann insbesondere vorgesehen sein, dass als Metall elementares Silber eingesetzt wird. Das Metall kann als Beschichtung auf dem Trägermaterial angeordnet sein oder in den Träger einimprägniert werden.

Weiterhin umfasst die vorliegende Erfindung eine Wundauflage, die eine Abdeckschicht und eine Wundkontaktschicht umfasst. Die Wundkontaktschicht umfasst dabei eine Zusammensetzung oder eine Wundsalbe umfassend eine hydrophile Basis in die Hydrocolloide dispergiert sind, wobei die hydrophile Basis mindestens einen Emulgator umfasst. Weiterhin umfasst die Wundkontaktschicht dabei eine Zusammensetzung oder eine Wundsalbe umfassend eine hydrophile Basis in die Hydrokolloide dispergiert sind, die mindestens eine freisetzbare wundheilungsfördernde Substanz umfassen, wobei die hydrophile Basis mindestens einen Emulgator umfasst. Insbesondere umfasst die vorliegende Erfindung eine Wundauflage gemäß Anspruch 13, die eine Abdeckschicht und eine Wundkontaktschicht umfasst, wobei die Wundkontaktschicht eine medizinische Zusammensetzung aufweist, die 50 bis 99 Gew.-% einer hydrophilen Basis umfasst, in die 1 bis 50 Gew.-% Hydrokolloide dispergiert sind, die mindestens eine freisetzbare wundheilungsfördernde Substanz umfassen, wobei die hydrophile Basis zu 0,5 bis 50 Gew.-% mindestens einen Emulgator umfasst. Insbesondere umfasst die vorliegende Erfindung eine Wundauflage gemäß Anspruch 13, die eine Abdeckschicht und eine Wundkontaktschicht umfasst, wobei die Wundkontaktschicht eine Zusammensetzung mit weniger als 10 Gew.-% Wasser umfasst, die 60 bis 95 Gew.-% einer hydrophilen Basis aufweist, in die 5 bis 40 Gew.-% Hydrocolloide dispergiert sind, wobei die hydrophile Basis zu 0,5 bis 50 Gew.-% mindestens einen Emulgator umfasst. Hierbei kann eine erfindungsgemäße Wundauflage weiterhin alle bevorzugten Eigenschaften oder Merkmale insbesondere der oben beschriebenen Wundkontaktschicht als auch der oben beschriebenen Zusammensetzung aufweisen. Insbesondere umfasst die vorliegende Erfindung eine Wundauflage gemäß Anspruch 13, die eine Abdeckschicht und eine Wundkontaktschicht umfasst, wobei die Wundkontaktschicht ein Trägermaterial und eine Zusammensetzung mit weniger als 10 Gew.-% Wasser umfasst, die weiterhin 60 bis 95 Gew.-% einer hydrophilen Basis, in die 5 bis 40 Gew.-% Hydrocolloide dispergiert sind, umfasst, wobei die hydrophile Basis zu 0,5 bis 50 Gew.-% mindestens einen Emulgator umfasst.

Gemäß einer Weiterbildung der Erfindung umfasst die vorliegende Erfindung auch eine Wundauflage gemäß Anspruch 13, die eine Abdeckschicht, eine absorbierende Schicht und eine Wundkontaktschicht umfasst, wobei die Wundkontaktschicht eine Zusammensetzung mit weniger als 10 Gew.-% Wasser umfasst, die 60 bis 95 Gew.-% einer hydrophile Basis mit 0,5 bis 50 Gew.-% an mindestens einem Emulgator umfasst, in die 5 bis 40 Gew.-% Hydrocolloide dispergiert sind.

Gemäß einer Weiterbildung der Erfindung umfasst die vorliegende Erfindung auch eine Wundauflage gemäß Anspruch 13, die eine Abdeckschicht, eine absorbierende Schicht und eine Wundkontaktschicht umfasst, wobei die Wundkontaktschicht eine Zusammensetzung mit weniger als 10 Gew.-% Wasser umfasst, die 50 bis 99 Gew.-% einer hydrophile Basis mit 0,5 bis 50 Gew.-% an mindestens einem Emulgator umfasst, in die 1 bis 50 Gew.-% Hydrocolloide dispergiert sind, die mindestens eine freisetzbare wundheilungsfördernde Substanz umfassen.

Als Abdeckschicht kann eine Wundauflage insbesondere eine Polymerfolie oder einen Polymerfilm aufweisen. Ganz besonders bevorzugt sind Polymerfilme, die eine hohe Wasserdampfdurchlässigkeit aufweisen. Hierzu sind besonders Polyurethan-, Polyetherurethan-, Polyesterurethan-, Polyether-Polyamid-Copolymer-, Polyacrylat- oder Polymethacrylat-Filme geeignet. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 bis 50 µm, insbesondere 20 bis 40 µm und ganz besonders bevorzugt von 25 bis 30 µm aufweisen. Die Wasserdampfdurchlässigkeit des Polymerfilms der Wundauflage weist vorzugsweise mindestens 750 g/ m²/24 Std., insbesondere mindestens 1000 g/ m²/24 Std. und ganz besonders bevorzugt mindestens 2000 g/ m²/ 24 Std. auf (gemessen nach DIN 13726).

Darüber hinaus kann vorgesehen sein, dass eine erfindungsgemäße Wundauflage als so genanntes Inseldressing vorliegt. Hierbei weist die Wundkontaktschicht eine kleinere Auflagefläche auf als die Abdeckschicht, d.h. die Wundkontaktschicht ist entlang ihres Umfangs von einer Abdeckschicht umgeben. Dabei kann die Abdeckschicht ein Haftklebemittel aufweisen oder klebend ausgerüstet sein, so dass die gesamte Wundauflage auf der Haut eines Patienten haften oder kleben kann. Dieser Auftrag des Haftklebemittels kann sowohl vollflächig als auch diskontinuierlich oder nur in bestimmten Bereichen erfolgen. Bei dem verwendeten Klebemittel kann es sich um einen üblichen Haftkleber, insbesondere um einen Acrylathaftkleber oder einen druckempfindlichen Haftkleber auf Basis von Polyurethanen handeln. Bevorzugt handelt es sich um Gelhaftkleber, insbesondere auf Basis von Polyurethanen, insbesondere wässrigen Polyurethanen. Ganz besonders bevorzugt handelt es sich um Hydrogelhaftkleber, insbesondere auf Basis von wässrigen Acrylaten.

Gemäß einer Weiterführung der Erfindung kann die Wundauflage eine Abdeckschicht aufweisen, die vollflächig mit einem Haftklebemittel beschichtet ist. Die Wasserdampfdurchlässigkeit dieser mit dem Haftklebemittel versehenen Trägerschicht beträgt dabei vorzugsweise mindestens 1000 g/ m²/24 Std., besonders bevorzugt mindestens 1200 g/ m²/24 Std. und ganz besonders bevorzugt mindestens 2000 g/ m²/ 24 Std. (gemessen nach DIN EN 13726).

Eine erfindungsgemäße Wundauflage kann in jeder geometrischen Form beispielsweise in dreieckiger, runder, ovaler oder quadratischer, rechteckiger oder jeder symmetrischen oder unsymmetrischen Form vorliegen.

Es kann weiterhin vorgesehen sein, das eine erfindungsgemäße Wundauflage weitere Schichten aufweist, die unterschiedliche Funktionen besitzen können. Gemäß einer Weiterentwicklung der vorliegenden Erfindung weist die Wundauflage mindestens eine weitere Schicht auf. Diese Schicht kann bevorzugt eine Releaseschicht zum Schutz vor Verschmutzungen sein, die auf der im anwendungsgerechten Zustand der Wundauflage auf der Wunde zu liegende Seite der Wundkontaktschicht aufgebracht ist. Es kann auch vorgesehen sein, dass die Wundauflage mindestens eine weitere Schicht zwischen der Wundkontaktschicht und der Abdeckschicht aufweist. Diese weitere Schicht kann eine absorbierende Schicht wie beispielsweise eine absorbierende Schicht aus einem hydrophilen Schaummaterial aus beispielsweise Polyurethan sein.

Damit ist auch die Verwendung einer medizinischen Zusammensetzung gemäß Anspruch 15, zur Herstellung einer Wundkontaktschicht oder einer Wundauflage insbesondere zur Behandlung von Bandwunden oder chronischen Wunden Gegenstand der vorliegenden Erfindung.

In einer besonderen Ausgestaltung der Erfindung ist vorgesehen, dass eine erfindungsgemäße Wundauflage in einer Verpackung angeordnet ist. Insbesondere ist dabei vorgesehen, dass die Verpackung eine sterile Verpackung ist. In einer weiteren besonderen Ausgestaltung der Erfindung ist vorgesehen, dass ein System umfassend eine Wundkontaktschicht der beschriebenen Art und eine separate Wundauflage in einer Verpackung angeordnet sind. Insbesondere ist dabei auch vorgesehen, dass die Verpackung eine sterile Verpackung ist. In einer besonders bevorzugten Ausführung dieses Systems liegt in der Verpackung oder in der sterilen Verpackung jeder einzelne Bestandteil oder jede Gruppe von Bestandteilen jeweils separat in Unterverpackungen vor. Es kann auch vorgesehen sein, dass jede Unterverpackung eine sterile Unterverpackung ist.

Nachstehend wird die Erfindung unter Bezugnahme auf die Zeichnungen und die Beispiele erläutert. In den Zeichnungen zeigt:
Figur 1: Wundkontaktschicht im Querschnitt
Figur 2: Wundauflage im Querschnitt
Figur 3: alternative Wundauflage im Querschnitt

### Beispiele

### 1) Zusammensetzung 1

| Nr. | Handelsname | Bezeichnung - INCI, Funktion | Gehalt Gew.-% |
|---|---|---|---|
| 1 | IMWITOR 780 K (Fa. Sasol, Witten - Deutschland) | Isostearyl Diglyceryl Succinate, nichtionischer W/O-Emulgator HLB 3,7 | 5,0 |
| 2 | IMWITOR 900 K (Fa. Sasol, Witten - Deutschland) | Glyceryl Stearate, Co-Emulgator | 4,0 |
| 3 | SOFTISAN 100 (Fa. Sasol, Witten - Deutschland) | Hydrogenated Coco-Glycerides, polares Fett | 4,0 |
| 4 | SOFTISAN 378 (Fa. Sasol, Witten - Deutschland) | Caprylic/Capric/Myristic/Stearic Triglyceride, polares Fett | 23,0 |
| 5 | SOFTISAN 649 (Fa. Sasol, Witten - Deutschland) | Bis-Diglyceryl Polyacyladipate-2, polares Fett | 19,0 |
| 6 | MERKUR Vaseline 115 (Fa. Merkur Vaseline GmbH & Co. KG, Hamburg - Deutschland) | Petrolatum, unpolares Lipid | 25,0 |
| 7 | Aquacel®Ag Fa. Convatec Vertriebs-GmbH, München - Deutschland) | Natiumcarboxymethylcellulose-Faser mit Silberionen | 20,0 |

### Herstellung der Zusammensetzung 1:

Phase A (Komponenten 1 bis 6) wird bei ca. 75-80°C geschmolzen und verrührt.
Anschließend wird Phase B (Komponente 7, Fasern lagen als Einzelfasern mit einer Länge von 2 -10 mm vor) unter starkem Rühren in Phase A dispergiert. Die Salbenmasse wird unter starkem Rühren abgekühlt, so dass eine feine Kristallstruktur entsteht. Der Tropfpunkt der Zusammensetzung beträgt 46 °C (bestimmt nach Ph. Eur. 2002, Methode 2.2.17).

### 2) Zusammensetzung 2

| Nr. | Handelsname (Hersteller) | Bezeichnung - INCI, Funktion | Gehalt Gew.-% |
|---|---|---|---|
| 1 | IMWITOR 377 (Fa. Sasol, Witten - Deutschland) | Glyceryl Laurate Citrate, ionischer O/W-Emulgator | 5,0 |
| 2 | IMWITOR 900 K (Fa. Sasol, Witten - Deutschland) | Glyceryl Stearate, Co-Emulgator | 4,0 |
| 3 | SOFTISAN 100 (Fa. Sasol, Witten - Deutschland) | Hydrogenated Coco-Glycerides, polares Fett | 4,0 |
| 4 | SOFTISAN 378 (Fa. Sasol, Witten - Deutschland) | Caprylic/Capric/Myristic/Stearic Triglyceride, polares Fett | 23,0 |
| 5 | SOFTISAN 649 (Fa. Sasol, Witten - Deutschland) | Bis-Diglyceryl Polyacyladipate-2, polares Fett | 19,0 |
| 6 | MERKUR Vaseline 115 (Fa. Merkur Vaseline GmbH & Co. KG, Hamburg - Deutschland) | Petrolatum, unpolares Lipid | 25,0 |
| 7 | Aquacel®Ag Fa. Convatec Vertriebs-GmbH, München - Deutschland) | Natiumcarboxymethylcellulose-Faser mit Silberionen | 20,0 |

### Herstellung der Zusammensetzung 2:

Phase A (Komponenten 1 bis 6) wird bei ca. 75-80°C geschmolzen und verrührt.
Anschließend wird Phase B (Komponente 7, Fasern lagen als Einzelfasern mit einer Länge von 2 -10 mm vor) unter starkem Rühren in Phase A dispergiert. Die Salbenmasse wird unter starkem Rühren abgekühlt, so dass eine feine Kristallstruktur entsteht. Der Tropfpunkt der Zusammensetzung beträgt 48 °C (bestimmt nach Ph. Eur. 2002, Methode 2.2.17).

### 3) Wundkontaktschicht 1

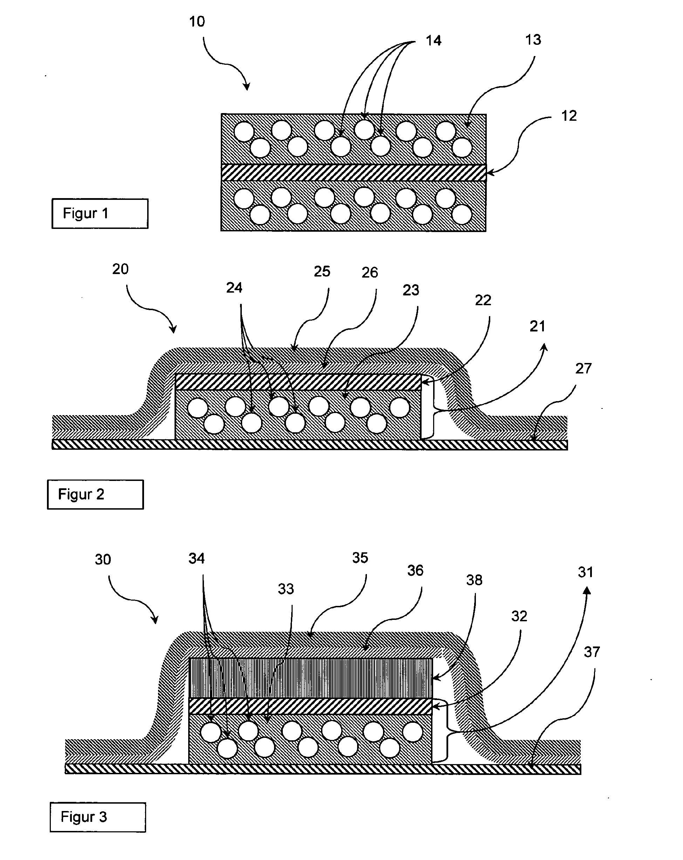

Die vorliegende Wundkontaktschicht hat den mit Figur 1 wiedergegebenen Aufbau. Demnach weist die Wundkontaktschicht (10) ein Trägermaterial (1) aus einem hydrophoben 100 %-igen Polyester-Gewirk (Fa. Theodor Preuss GmbH & Co. KG, Ubstadt-Weiher - Deutschland) auf, das auf beiden Seiten bzw. Oberflächen mit einer erfindungsgemäßen Zusammensetzung (2a und 2b) analog Beispiel 1 bestrichen ist. Der Unterschied besteht darin, dass statt silberdotierten CMC-Fasern silberdotierte CMC-Partikel mit einer Partikelgröße von durchschnittlich 300 µm verwendet wurden. Die Zusammensetzung besteht demnach aus einer hydrophilen Basis (13) analog Beispiel 1 und CMC-Partikel (14), die mit freisetzbaren Silberionen in einer Menge von 10 Gew.-% bezogen auf die Menge an Partikeln beladen sind. Die Zusammensetzung benetzt vollständig das Trägermaterial, wobei die Auftragsmenge auf jeder Seite jeweils 200 g/ m² beträgt. Das Trägergewirk hat ein Flächengewicht von 63 g/ m² (ungedehnt) und weist ca. 40 sechseckigen Öffnungen pro 100 cm auf (in Figur 1 nicht dargestellt). Die maximale lichte Weite der Öffnungen beträgt 2 mm. Die Wundkontaktschicht weist einen guten Zusammenhalt auf und lässt sich besonders gut auf einer zu behandelnden Wunde applizieren.

### 4) Wundkontaktschicht 2

Auch diese Wundkontaktschicht weist einen Aufbau wie mit Figur 1 auf. Bei dieser Wundkontaktschicht (10) weist die Zusammensetzung die mit Beispiel 1 angegebenen Bestandteile auf. Das Trägermaterial (1) besteht aus einem hydrophoben 100%-igen Polyamid-Gewirk (Fa. Theodor Preuss GmbH & Co. KG, Ubstadt-Weiher - Deutschland) mit einem Flächengewicht von ca. 90 g/ m² (ungedehnt) und weist ca. 46 sechseckigen Öffnungen pro 100 cm auf (in Figur 1 nicht dargestellt). Die maximale lichte Weite der Öffnungen beträgt 0,8 - 1,0 mm. Das Auftragsgewicht der Zusammensetzung beträgt 240 g/ m².

Die Beschichtung des textilen Trägermaterials mit der hydrophilen Zusammensetzung erfolgt, indem das Trägermaterial über eine Umlenkwalze durch ein warmes Vorratsbad (40 °C) der hydrophilen Zusammensetzung 1, 2 oder einer analogen Zusammensetzung mit silberdotierten CMC-Partikeln geführt wird. Nach dem Durchlaufen des Bades wird die überschüssige Menge an übertragener Zusammensetzung mit Hilfe einer Quetschwalze abgestreift. Das beschichtete Material wird auf Raumtemperatur gebracht, konfektioniert, verpackt und sterilisiert.

### 5) Wundkontaktschicht 3

Auch diese Wundkontaktschicht weist einen Aufbau wie mit Figur 1 auf. Bei dieser Wundkontaktschicht (10) weist die Zusammensetzung die mit Beispiel 2 angegebenen Bestandteile auf. Das Trägermaterial (1) besteht aus einem hydrophoben 100%-igen Polyamid-Gewirk (Fa. Theodor Preuss GmbH & Co. KG, Ubstadt-Weiher - Deutschland) mit einem Flächengewicht von ca. 80 g/ m² (ungedehnt) und weist ca. 40 sechseckigen Öffnungen pro 100 cm auf (in Figur 1 nicht dargestellt). Die maximale lichte Weite der Öffnungen beträgt 1,2 - 1,5 mm. Das Auftragsgewicht der Zusammensetzung beträgt ca. 330 g/ m².

Die Beschichtung des textilen Trägermaterials mit der hydrophilen Zusammensetzung erfolgt, indem das Trägermaterial über eine Umlenkwalze durch ein warmes Vorratsbad (60 °C) der hydrophilen Zusammensetzung 2 geführt wird. Nach dem Durchlaufen des Bades wird die überschüssige Menge an übertragener Zusammensetzung mit Hilfe einer Quetschwalze abgestreift. Das beschichtete Material wird auf Raumtemperatur gebracht, konfektioniert, verpackt und sterilisiert.

### 6) Wundauflage 1

Mit Figur 2 ist eine erfindungsgemäße Wundauflage (20) als so genanntes Inseldressing wiedergegeben. Die Wundauflage besteht aus einer Abdeckschicht (24), und einer Wundkontaktschicht (21). Die Wundkontaktschicht ihrerseits besteht aus einem Trägermaterial (22), das ein hydrophobes Nonwoven aus Polyesterfasern ist, das mit einer Zusammensetzung (23) laut Beispiel 2 analogen Zusammensetzung bestrichen ist. Der Unterschied besteht darin, das hier silberdotierte Alginatpartikel verwendet werden. Die Zusammensetzung besteht demnach aus einer hydrophilen Basis (23) in die Alginat-Partikel (24) dispergiert sind, die mit freisetzbarem ionischen Silber beladen sind. Die Zusammensetzung überdeckt mit einer Auftragsmenge von 180 g/ m² vollständig das Polyester-Nonwoven (wasserstrahlverfestigt, Flächengewicht 50 g/ m²). Die Wundkontaktschicht ist mit einer Abdeckschicht (24) überdeckt, die mit einem Polyacrylat-Haftklebemittel (25) vollflächigen beschichtet ist. Die Abdeckschicht ist ein 30 µm dicker Polyurethanfilm mit einer Wasserdampfdurchlässigkeit von 1100 g/ m² / 24 Std., die allseitig über die Umfangsgrenzen der Wundkontaktschicht hinaus reicht, so dass mittels der klebenden Ränder der Abdeckschicht (26a, 26b) die Wundkontaktschicht auf der Haut eines Patienten befestigt werden kann. Zugleich ist die Wundkontaktschicht (22) mittels des Haftklebers (25) auf der Abdeckschicht fixiert.

### 7) Wundauflage 2

Mit Figur 3 ist eine weitere erfindungsgemäße Wundauflage (30) gezeigt, die gegenüber der mit Figur 2 gezeigten Wundauflage (20) eine zusätzliche absorbierende Schicht (37) aufweist. Diese zusätzliche absorbierende Schicht (37) besteht aus einem hydrophilen, offenzelligen Polyurethanschaum mit einem Flächengewicht von 500 g/ m² bei einer Dicke von 5 mm. Die absorbierende Schicht ist mittels der Haftklebeschicht (35) aus einem Acrylat-Dispersionshaftkleber auf der Abdeckschicht fixiert. Die Abdeckschicht besteht aus einem Polyurethanfilm mit einer Dicke von 25 µm und einer Wasserdampfdurchlässigkeit von 1200 g/ m²/ 24 Std.. Die Wundkontaktschicht (31) besteht aus einem Polyamid-Gewirk (32), das mit einer Zusammensetzung (33) laut Beispiel 1 (150 g/ m²) beschichtet ist. Das Polyamid-Gewirk (Fa. Theodor Preuss GmbH & Co. KG, Ubstadt-Weiher - Deutschland) weist 45 sechseckige Öffnungen pro 100 cm auf (in Figur 3 nicht dargestellt). Die maximale lichte Weite der Öffnungen beträgt 0,8 bis 1,0 mm. Das Flächengewicht beträgt 86 g/ m². Die Wundauflage ist besonders zum Einsatz bei stark nässenden Wunden einzusetzen. Die Wundauflage pflegt durch den Anteil an Triglyceriden in der Zusammensetzung die periphere Wundhaut und zeigt auch bei längerem Gebrauch keine Wundverklebung.

## Patentansprüche

1. Medizinische Zusammensetzung zur Wundbehandlung umfassend 50 bis 99 Gew.-% hydrophile Basis, in die 1 bis 50 Gew.-% Hydrocolloide dispergiert sind, wobei die hydrophile Basis 0,5 bis 50 Gew.-% mindestens eines Emulgators umfasst, und wobei die Hydrocolloide mindestens eine freisetzbare, wundheilungsfördernde Substanz umfassen, **dadurch gekennzeichnet, dass**
- die Zusammensetzung weniger als 10 Gew.-% Wasser enthält, und dass
- die hydrophile Basis weiterhin 20 bis 80 Gew.-% Mono-, Di- und/ oder Triglyceriden und/ oder Voll- oder Partialester Oligomerer des Glycerins umfasst.

2. Zusammensetzung nach Anspruch 1 oder 2 **dadurch gekennzeichnet, dass** die hydrophile Basis eine Creme, Cremegrundlage oder Salbe ist.

3. Zusammensetzung nach mindestens einem der vorgenannten Ansprüche **dadurch gekennzeichnet, dass** die Hydrocolloide in Partikelform vorliegen und die Partikel einen Wassergehalt von weniger als 10 Gew.-% bezogen auf das Gewicht der Hydrocolloidpartikel aufweisen.

4. Zusammensetzung nach mindestens einem der vorgenannten Ansprüche **dadurch gekennzeichnet, dass** die Hydrocolloide ausgewählt werden aus der Gruppe der Cellulose oder Derivate oder deren Salze sowie Alginsäure oder deren Derivate oder Salze sowie der Polyacrylate oder deren Derivate oder Salze.

5. Medizinische Zusammensetzung nach mindestens einem der vorgenannten Ansprüche, **dadurch gekennzeichnet, dass** die wundheilungsfördernde Substanz ausgewählt wird aus der Gruppe der Vitamine oder Provitamine, Carotinoide, Analgetika, Antiseptika, Hämostyptika, Antihistaminika, antimikrobiellen Metalle oder deren Salze, pflanzlichen wundheilungsfördernden Substanzen oder Substanzgemische, Pflanzenextrakte, Enzyme, Wachstumsfaktoren, Enzyminhibitoren sowie Kombinationen hiervon.

6. Zusammensetzung nach mindestens einem der vorgenannten Ansprüche **dadurch gekennzeichnet, dass** die hydrophile Basis weiterhin 10 bis 30 Gew.-% unpolare Lipide aus der Gruppe Vaseline, Petrolatum, Parafinöl oder Waxe umfasst.

7. Zusammensetzung nach mindestens einem der vorgenannten Ansprüche **dadurch gekennzeichnet, dass** der Emulgator ein ionischer O/W-Emulgator oder ein nichtionischer W/O-Emulgator ist.

8. Wundsalbe umfassend eine Zusammensetzung gemäß mindestens einem der vorgenannten Ansprüche 1 bis 7.

9. Wundkontaktschicht (10, 21, 31) umfassend eine Zusammensetzung gemäß einem der vorgenannten Ansprüche 1 bis 7 oder einer Wundsalbe gemäß Anspruch 8 und ein Trägermaterial (1) für die Zusammensetzung oder die Wundsalbe.

10. Wundkontaktschicht (10, 21, 31) nach Anspruch 9 **dadurch gekennzeichnet, dass** das Trägermaterial (1) ein Nonwoven, Gestrick, Gewirk oder Gewebe ist.

11. Wundkontaktschicht (10, 21, 31) nach mindestens einem der vorgenannten Ansprüche 9 oder 10 **dadurch gekennzeichnet, dass** das Trägermaterial (1) aus einem hydrophoben Gestrick, Gewirk oder Gewebe besteht.

12. Wundkontaktschicht (10, 21, 31) nach mindestens einem der vorgenannten Ansprüche 9 bis 11 **dadurch gekennzeichnet, dass** das Trägermaterial (1) ein Polyamid-Gewirk umfasst.

13. Wundauflage (20, 30) umfassend eine Wundkontaktschicht (10, 21, 31) nach mindestens einem der Ansprüche 9 bis 12 und eine Abdeckschicht (24, 34).

14. Wundauflage (20, 20) gemäß Anspruch 13 **dadurch gekennzeichnet, dass** die Wundauflage weiterhin eine absorbierende Schicht (37), insbesondere einen hydrophilen Polyurethan-Schaum umfasst, die der Wundkontaktschicht (10, 21, 31) benachbart ist.

15. Verwendung einer Zusammensetzung gemäß einem der vorgenannten Ansprüche 1 bis 7 zur Herstellung einer Wundsalbe, einer Wundkontaktschicht oder einer Wundauflage zur Behandlung von Wunden insbesondere Brandwunden oder chronischen Wunden.

## Claims

1. Medicinal composition for wound treatment comprising 50 to 99 wt% of a hydrophilic base whereto 1 to 50 wt% of hydrocolloids is dispersed, wherein the hydrophilic base comprises 0.5 to 50 wt% of at least one emulsifier, and wherein the hydrocolloids comprise at least one releasable wound healing promoter substance, **characterized in that**
- the composition contains less than 10 wt% of water, and **in that**
- the hydrophilic base further comprises 20 to 80 wt% of mono-, di- and/or triglycerides and/or full or partial esters of glycerol oligomers.

2. Composition according to Claim 1, **characterized in that** the hydrophilic base is a cream, a cream base or an ointment.

3. Composition according to at least one of the aforementioned claims, **characterized in that** the hydrocolloids are in particle form and the particles have a water content of less than 10 wt% based on the weight of the hydrocolloid particles.

4. Composition according to at least one of the aforementioned claims, **characterized in that** the hydrocolloids are selected from the group of cellulose or derivatives or their salts and also alginic acid or its derivatives or salts and also polyacrylates or their derivatives or salts.

5. Medicinal composition according to at least one of the aforementioned claims, **characterized in that** the wound healing promoter substance is selected from the group of vitamins or provitamins, carotenoids, analgesics, antiseptics, haemostyptics, antihistamines, antimicrobial metals or their salts, plant-derived wound healing promoter substances or substance mixtures, plant extracts, enzymes, growth factors, enzyme inhibitors and combinations thereof.

6. Composition according to at least one of the aforementioned claims, **characterized in that** the hydrophilic base further comprises 10 to 30 wt% of apolar lipids from the group consisting of Vaseline, petrolatum, paraffin oil and waxes.

7. Composition according to at least one of the aforementioned claims, **characterized in that** the emulsifier is an anionic O/W emulsifier or a nonionic W/O emulsifier.

8. Wound ointment comprising a composition according to at least one of aforementioned Claims 1 to 7.

9. Wound contact layer (10, 21, 31) comprising a composition according to any of aforementioned Claims 1 to 7 or a wound ointment according to Claim 8 and a carrier material (1) for the composition or the wound ointment.

10. Wound contact layer (10, 21, 31) according to Claim 9, **characterized in that** the carrier material (1) is a nonwoven, a knitted fabric or a woven fabric.

11. Wound contact layer (10, 21, 31) according to at least one of aforementioned Claims 9 and 10, **characterized in that** the carrier material (1) consists of a hydrophobic knitted or woven fabric.

12. Wound contact layer (10, 21, 31) according to at least one of aforementioned Claims 9 to 11, **characterized in that** the carrier material (1) comprises a knitted polyamide fabric produced on a machine without independently movable needles.

13. Wound dressing (20, 30) comprising a wound contact layer (10, 21, 31) according to at least one of Claims 9 to 12 and a cover layer (24, 34).

14. Wound dressing (20, 30) according to Claim 13, **characterized in that** the wound dressing further comprises an absorbent layer (37), in particular a hydrophilic polyurethane foam, adjacent to the wound contact layer (10, 21, 31).

15. Use of a composition according to any of aforementioned Claims 1 to 7 in the manufacture of a wound ointment, of a wound contact layer or of a wound dressing for treatment of wounds, in particular burn wounds or chronic wounds.

## Revendications

1. Composition médicinale pour le traitement de plaies, comprenant 50 à 99 % en poids de base hydrophile, dans laquelle 1 à 50 % en poids d'hydrocolloïdes sont dispersés, la base hydrophile comprenant 0,5 à 50 % en poids d'au moins un émulsifiant, et les hydrocolloïdes comprenant au moins une substance libérable, favorisant la cicatrisation, **caractérisée en ce que**
- la composition contient moins de 10 % en poids d'eau, et **en ce que**
- la base hydrophile comprend en outre 20 à 80 % en poids de mono-, di- et/ou triglycérides et/ou d'esters entiers ou partiels d'oligomères de glycérine.

2. Composition selon la revendication 1, **caractérisée en ce que** la base hydrophile est une crème, une base de crème ou un onguent.

3. Composition selon au moins l'une quelconque des revendications précédentes, **caractérisée en ce que** les hydrocolloïdes se présentent sous forme particulaire et les particules présentent une teneur en eau de moins de 10 % en poids, par rapport au poids des particules d'hydrocolloïdes.

4. Composition selon au moins l'une quelconque des revendications précédentes, **caractérisée en ce que** les hydrocolloïdes sont choisis dans le groupe constitué par la cellulose ou ses dérivés ou sels, ainsi que l'acide alginique ou ses dérivés ou sels, ainsi que les polyacrylates ou leurs dérivés ou sels.

5. Composition médicinale selon au moins l'une quelconque des revendications précédentes, **caractérisée en ce que** la substance favorisant la cicatrisation est choisie dans le groupe constitué par les vitamines ou les provitamines, les caroténoïdes, les analgésiques, les antiseptiques, les hémostatiques, les antihistaminiques, les métaux antimicrobiens ou leurs sels, les substances ou mélanges de substances végétales favorisant la cicatrisation, les extraits végétaux, les enzymes, les facteurs de croissance, les inhibiteurs enzymatiques, ainsi que leurs combinaisons.

6. Composition selon au moins l'une quelconque des revendications précédentes, **caractérisée en ce que** la base hydrophile comprend en outre 10 à 30 % en poids de lipides apolaires du groupe constitué par la vaseline, le pétrolatum, l'huile de paraffine ou les cires.

7. Composition selon au moins l'une quelconque des revendications précédentes, **caractérisée en ce que** l'émulsifiant est un émulsifiant H/E ionique ou un émulsifiant E/H non ionique.

8. Onguent vulnéraire comprenant une composition selon au moins l'une quelconque des revendications 1 à 7 précédentes.

9. Couche en contact avec la plaie (10, 21, 31), comprenant une composition selon l'une quelconque des revendications 1 à 7 précédentes ou un onguent vulnéraire selon la revendication 8 et un matériau support (1) pour la composition ou l'onguent vulnéraire.

10. Couche en contact avec la plaie (10, 21, 31) selon la revendication 9, **caractérisée en ce que** le matériau support (1) est un non-tissé, un tricot ou un tissu.

11. Couche en contact avec la plaie (10, 21, 31) selon au moins l'une quelconque des revendications 9 ou 10, **caractérisée en ce que** le matériau support (1) est constitué d'un tricot ou d'un tissu hydrophobe.

12. Couche en contact avec la plaie (10, 21, 31) selon au moins l'une quelconque des revendications 9 à 11 précédentes, **caractérisée en ce que** le matériau support (1) comprend un tricot en polyamide.

13. Pansement (20, 30) comprenant une couche en contact avec la plaie (10, 21, 31) selon au moins l'une quelconque des revendications 9 à 12 et une couche supérieure (24, 34).

14. Pansement (20, 30) selon la revendication 13, **caractérisé en ce que** le pansement comprend en outre une couche absorbante (37), notamment une mousse de polyuréthane hydrophile, qui est adjacente à la couche en contact avec la plaie (10, 21, 31).

15. Utilisation d'une composition selon l'une quelconque des revendications 1 à 7 précédentes pour la fabrication d'un onguent vulnéraire, d'une couche en contact avec la plaie ou d'un pansement pour le traitement de plaies, notamment de brûlures ou de plaies chroniques.
